# EUROPEAN PATENT APPLICATION

(11) **EP 2 395 088 A1**
(43) Date of publication of application: **14.12.2011**
(21) Application number: 10179712.4
(22) Date of filing: 24.09.2010
(51) Int. Cl.: C12N 15/82, C12N 9/10

(54) **A recombinant DNA molecule, vector, plant cell and plant expression cassette producing large amounts of functionally active cysteine synthase and use thereof**

(30) Priority: 25.09.2009 PL 38913109
(71) Applicant: Instytut Biochemii I Biofizyki Pan, 02-106 Warszawa (PL)
(72) Inventor: Liszewska, Frantz, 80000 Amiens (FR); Wawrzynska, Anna, 05-804 Pruszków (PL); Sirko, Agnieszka, 01-227 Warszawa (PL)
(74) Representative: Brodowska, Iwona

(57) **Abstract**

The present invention provides the means and the methods for obtaining plant materials producing large amounts of functionally active cysteine synthase (EC 2.5.1.47) that rely on an environment-friendly and health-safe enzyme production process for synthesis of L-cysteine and L-cystine, therefore suitably accounting among the c-GMP standards for production of the aforementioned compounds, namely of non-human and non-animal origin. Described is a recombinant DNA molecule comprising a nucleic acid molecule encoding a protein having cysteine synthase activity, wherein the said nucleic acid molecule is operably linked to regulatory elements allowing the expression of the nucleic acid molecule in plant cells. The present invention also provides a vector harboring the plant expression cassette comprising the said recombinant DNA molecule, as well as plant cells, plant tissues and plants transformed therewith. The present invention further describes the use of the aforementioned recombinant DNA molecule and vector in plant cell culture, plant tissue culture, and plant breeding; further presented are examples of the use of the said plant cells, plant tissues, plants, as well as the extracts from these plant raw materials for biosynthetic production of L-cysteine and/or L-cystine compounds being valuables in a broad range of applications, such as for production of additives or ingredients used in agroindustrial, cosmetic and pharmaceutic processes.

## Description

A recombinant DNA molecule, vector, plant cell and plant expression cassette producing large amounts of functionally active cysteine synthase and use thereof

### Technical Field

The present invention comprising recombinant DNA molecule, vector, plant cell and plant expression cassette containing a nucleic acid molecule encoding a protein having cysteine synthase activity (EC 2.5.1.47) from a prokaryote or archaebacteria, wherein said nucleic acid molecule is operably linked to regulatory elements allowing the expression of said the nucleic acid molecule in a plant cell.

### Background Art

The present invention provides the means and the methods for obtaining plant materials producing large amounts of functionally active cysteine synthase (EC 2.5.1.47) that rely to an environment-friendly and health-safe enzyme-using process for synthesis of L-cysteine and L-cystine, therefore suitably accounting among the c-GMP standards for production of the aforementioned compounds, i.e. as issued from non-human and non-animal origin; described is a recombinant DNA molecule comprising a nucleic acid molecule encoding a protein having cysteine synthase activity, wherein the said nucleic acid molecule is operably linked to regulatory elements allowing the expression of the nucleic acid molecule in plant cells. The present invention also provides a vector harboring the plant expression cassette comprising the said recombinant DNA molecule, as well as plant cells, plant tissues and plants transformed therewith. The present invention further describes the use of the aforementioned recombinant DNA molecule and vector in plant cell culture, plant tissue culture, and plant breeding; further presented are examples of the use of the said plant cells, plant tissues, plants, as well as the extracts from these plant raw materials for biosynthetic production of L-cysteine and/or L-cystine compounds being valuables in a broad range of applications, such as for production of additives or ingredients used in agroindustrial, cosmetic and pharmaceutic processes.

Currently three methods are used to produce cysteine: (1) purification from hair or feathers after dissolution in acid, (2) purification from liquid microorganism culture, (3) chemical synthesis used and described by Ajinomoto company.

There are publications from different research groups saying that larger amount of cysteine in transgenic plants can be gained by additional expression of plant genes encoding cysteine synthase. In most cases these proteins were directed to cytosol.

Unexpectedly the inventors found out in their own research that targeting of bacterial cysteine synthase to organelles, especially chloroplasts, according to solution presented in hereby invention, leads to about 1000 times increase of enzymatic cysteine synthase activity in plants containing the protein.

### Summary of invention

A recombinant DNA molecule comprising a nucleic acid molecule encoding a protein having cysteine synthase activity (EC 2.5.1.47) from a prokaryote or archaebacteria, wherein said nucleic acid molecule is operably linked to regulatory elements allowing the expression of said the nucleic acid molecule in a plant cell,

According to present invention, preferably, the recombinant DNA molecule, having cysteine synthase activity is called either of the followings: cysteine synthase (CS), *O-*acetylserine(thiol)lyase (OASTL), O -acetylserine sulfhydrylase (OASS),

Preferably, according to present invention, the recombinant DNA molecule, is operably linked to a nucleotide sequence encoding a transit peptide capable of directing the protein into a desired cellular compartment,

Preferably, according to present invention, the recombinant DNA molecule, wherein said cellular compartment is a plastid.

Preferably, according to present invention, the recombinant DNA molecule, having said regulatory elements, comprise a promoter active in a plant cell.

Preferably, according to present invention, the recombinant DNA molecule, wherein said promoter is inducible, constitutively expressed and/or is a cell, tissue or organ specific promoter,

The recombinant DNA molecule, wherein said promoter is tuber specific, seed-specific, endosperm-specific, embryo-specific, or phloem specific,

The recombinant DNA molecule of claim 1, comprising a nucleic acid molecule encoding a protein having cysteine synthase activity and derived either from *Escherichia colicysK* gene or its homologues from other bacteria, wherein said nucleic acid molecule is linked to regulatory elements allowing the expression of said the nucleic acid molecule in to a plant cell, preferably in to chloroplast.

A vector containing a plant expression cassette comprising at least one recombinant DNA molecule according to present invention.

A method for producing functionally active cysteine synthase in transgenic plant, plant tissue or plant cell comprising the introduction of at least one recombinant DNA molecule according to present invention or the vector according to present invention into a plant, plant tissue or plant cell,

A transgenic plant cell comprising stably integrated into its genome at least one recombinant DNA molecule according to present invention or a vector according to present invention, obtainable by the method according to present invention.

The transgenic plant cell or plant cell culture, wherein the amount of functionally active cysteine synthase is several-fold increased while comparing with that in plant cell breeding of the wild-type cultivar.

A transgenic plant or plant tissue comprising transiently or stably integrated into its genome at least one recombinant DNA molecule according to present invention, obtainable by the method according to present invention, or a transgenic plant obtained from at least one plant cell according to present invention.

The transgenic plant or plant tissue according to present invention, wherein the amount of functionally active cysteine synthase is several-fold increased while comparing with that in plant of the wild-type cultivar,

Harvestable plant tissue, comprising plant cells according to present invention.

Propagation material from a transgenic plant cell, plant tissue or plant according to present invention.

Use of a nucleic acid molecule according to present invention or at least one recombinant DNA molecule or a vector, a method, a plant cell, a plant or plant tissue, harvestable parts and propagation material according to present invention for production of transgenic plant material according to present invention.

Use of plant material or plant extract issued according to present invention for biochemical production of L-cysteine, L-cysteine-containing oligopeptide(s), or sulfhydryl-containing derived compound(s).

Use of any one for production of biochemical compounds or for production of additives and/or ingredients,

Use of compounds, additives and/or ingredients as defined for production of food, feed, cosmetics or drugs,

Any compound, additive, ingredient, food, feed, cosmetic or drug therefore, obtained by any mean according to present invention.

Unexpectedly, significantly higher activity of enzymatic cysteine synthase is acquired when protein is targeted to chloroplasts instead of targeting the protein to the cytosol.

Presence of the OASTL-A protein (product of *cysK* gene *Escherichia coli*) was detected in transgenic plants by Western blot method using OASTL-A-specific antibodies and was confirmed with microscope research that proved that the protein is present in chloroplasts.

Enzymatic activity of cysteine synthase in plants containing OASTL-A protein in chloroplasts increased 1000 times. In our method to determine cysteine synthase activity we assay the amount of the product, that is cysteine, while OAS and Na₂S were used as substrates in this reaction.

To obtain plant protein extract containing the said enzyme and to use this extract to obtain cysteine, the well known, standard methods can be used. For example, extraction of proteins in 0,1M Tris, pH 7.5 from minced plant tissues frozen in liquid nitrogen. Afterward the enzyme is purified by any known method, especially with HPLC, from potentially harmful ingredients or compounds that could inhibit its activity.

The idea to use plant material as a source of enzyme for biosynthesis of cysteine is innovative and attractive. Most of all, we use plants therefore there is a possibility of application of the product (created cysteine) in healthy food, approved by vegetarians. Additionally, the extracts from edible plant should have GRAS (generally recognized as safe) status.

The matter of the present invention has been shown and demonstrated by the authors on the model plant *N. tabacum*; however transformation of some worldwide extensively cultivated crops with the claimed recombinant DNA molecule would also give them a clear economic advantage. Sugar beet being a good example where, according to agricultural processing, leaves are "harvested" separately from the roots,

### Description of embodiments

**Examples**

Example 1 : Construction of expression cassettes containing a recombinant DNA molecule designed for targeting of bacterial cysteine synthase to plant chloroplasts.

A chimeric gene construct was developed to overexpress the *Escherichia colicysK* gene in plant cells. The *cysK* gene encoding cysteine synthase was amplified by polymerase chain reaction (PCR) using genomic DNA of the EC1250 strain (Jagura-Burdzy & Hulanicka, 1981) of *E. coli*K-12 as a template and the following pair of primers : 5'-cgg gat cca tga gta aga ttt ttg aag-3' and 5'-gcg gta ccg ctg gca tta ctg ttg c-3'. These primers introduced a *BamHI* site upstream of the *cysK* translation start codon and a *KpnI* site 7 nt downstream of the stop codon. The 1.0 kb PCR-amplified fragment was cloned after digestion with *BamHI* and *KpnI* into pUCRbcSL harboring a fragment of the tomato rbcS2 gene (Meier *et al.,* 1995) encoding a 57-amino acid transit peptide responsible for chloroplast targeting. The *Sall-Kpnl* insert from the resulting plasmid was then subcloned into pRTL2 (Carrington *et al.,* 1991) digested with *XhoI* and *KpnI,* i.e. between the 35S promotor and terminator of cauliflower mosaic virus (CaMV). The 2.0 kb *PstI-PstI* DNA fragment with the *cysK* plant expression cassette was further subcloned into plant expression binary vectors, either pGreen0029 or pGreen0229 (Hellens *et al.,* 2000) containing a selectable plant transformation marker, *npt II* or *bar,* providing resistance to kanamycin (kan) or phosphinotricin (ppt), respectively.

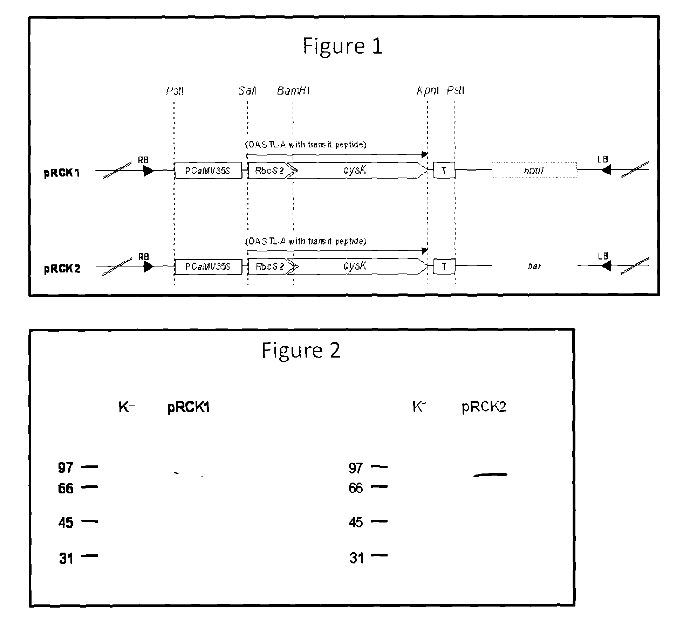

In Figure 1, there are shown schematic details of the T-DNA regions of two used binary plasmids with plant expression cassettes containing *cysK,* where cysK denotes the *E. coli* gene encoding OASTL-A, RbcS2- the sequence of tomato *RbcS2*gene encoding the transit peptide of the small subunit of Rubisco, PCaMV35S - the 35S promoter of CaMV (cauliflower mosaic virus) with dual enhancer, T- the CaMV transcription terminator, nptll- the gene encoding neomycin phosphotransferase, bar- the gene encoding phosphinothrycin acetyl-transferase, LB and RB- the left and right borders of the T-DNA.

Example 2 : *Agrobacterium-mediated* transformation of tobacco tissues and plants with the recombinant DNA molecule.

The plant expression constructs described in example 1 being based on the pGreen series vectors and therefore requiring a helper plasmid for replication into *Agrobacterium*, the pJIC_Soup plasmid (Hellens *et al.,* 2000) was first electroporated into cells of the *A. tumefaciens* LBA4404 strain (Hoekema *et al.,* 1983) ; then the recombinants plasmids were separately introduced by electroporation into the bacterial cells. Functionality of the plant expression construct described in example 1 was checked in tobacco at both, mRNA and protein levels. The assay of transient expression in planta of the *cysK* cassette was performed in leaf tissues of Nicotiana *benthamiana* plants grown in soil. The Northern blot, immunodetection and biochemical analysis were carried out using plant material collected at the two time point (72h and 96h after injection of the *N. benthamiana* leaves with the suspensions of the Agrobacterium cultures harboring the described plasmids). Presence of the heterologous *E. colicysK* mRNA transcript, immunodetection of *E. coli* OASTL-A protein and verification of elevated OASTL enzymatic activity in the leaf protein extracts revealed that the transgene can be efficiently expressed in plant cells. In Figure 2 there are shown results of immunodetection of OASTL-A after transient in planta expression of *E. coli cysK.* The left-hand panel shows the Western blot with protein extract from either non-injected leaves of *N. benthamiana* (K-) or leaves injected with Agrobacterium harboring the plasmid pRCK1 72 h post injection, while the right-hand panel shows the Western blot with protein extract from either non-injected leaves (K-) or leaves injected with *Agrobacterium* harboring the plasmid pRCK2 96h post injection. Bacterial OASTL-A was immunodetected using specific (anti-*E. coli* OASTL-A) polyclonal antibodies developed in rabbit. The positions of protein standards (in kDa) are shown.

Example 3 : Analysis of primary transformants and transgenic tobacco lines harboring the recombinant DNA molecule.

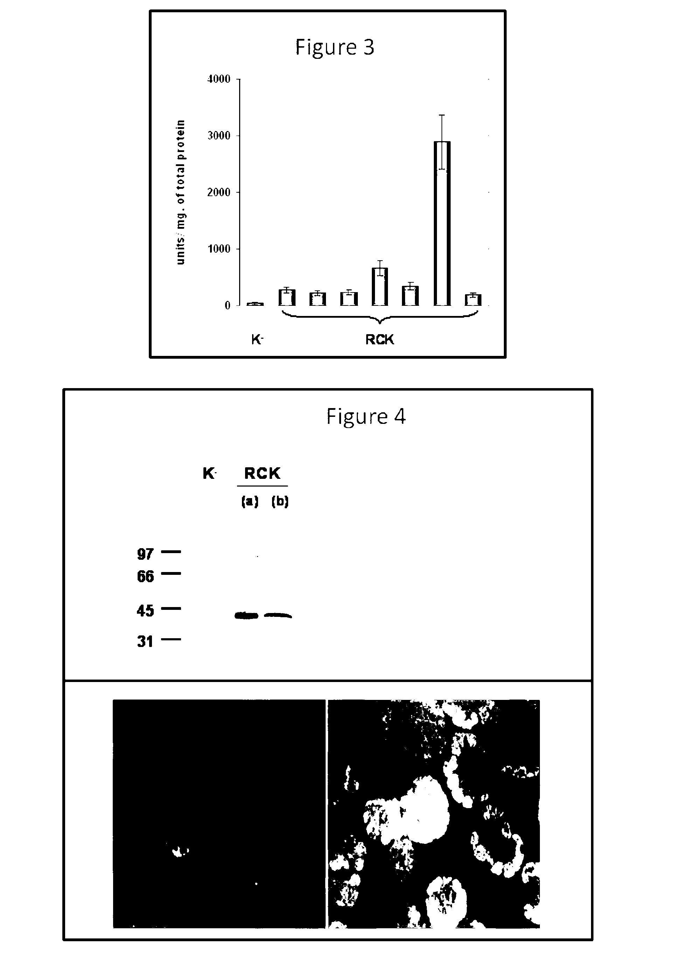

Tobacco *(nicotiana tabacum)* was chosen as a model plant for stable transformation because of the simplicity of its transformation method; the so called low alkaloid line LA Burley 21 (Legg *et al.,* 1970) was used all through further experiments. Presence of the OASTL-A protein in the regenerated tobacco plants was verified by immunodetection, and most of them had detectable amounts of the bacterial enzyme (16 plants out of 20 in the pRCK1 transformation experiment). The OASTL activity in extracts from the leaf tissues of transgenic plants was determined in a biochemical enzymatic assay according to a procedure adapted from the Gaitonde's method (Gaitonde, 1967; Warrilow & Hawkesford, 1998) : the enzymatic activity assayed in primary transformants at an early stage of plant development was increased up to about 70-fold in comparison to the parental wild-type line. In Figure 3, there are shown OAS-TL activity in primary transformants (T0). OASTL activity was assayed in leaf tissues of transgenic individuals at an early stage of vegetative development (8-week-old in vivo) by monitoring the formation of L-cysteine from OAS and sulfide after addition of 5 µl of the total protein extract from the leaves to 145 µl of the reaction mixture containing 100 mM Tris-HCl pH 7.5, 100 mM DTT, 8 mM OAS and 3 mM Na2S; the L-cysteine content in the mixture was measured spectrophotometrically, according to a procedure adapted from the Gaitonde's method. One unit of OASTL activity is defined as the amount of enzyme, which catalyzes the formation of 1 nmole of L-cysteine per minute under the conditions of the assay. Several transgenic lines were selected for further experiments. The criteria for the selection of the lines with stable and possibly highest expression of the transgene in the next generations were the following: the highest OASTL activity in the screened leaf tissue and the smallest number of inserts in the genome. The number of copies of the transgene that integrated into the plant genome is an important factor affecting the level and stability of expression; by genetic screening of the segregation of the resistance markers in the progeny, the copy number of the independent inserts containing the *cysK* transgene was evaluated for several transgenic plants producing bacterial OASTL-A. The observed segregation ratio (3:1) of marker-resistant (kanR or pptR) to marker-sensitive (kanS or pptS) plants in the progeny of transformants suggested that in these transgenics, T-DNA containing the *cysK* gene was inserted into a unique genetic locus.

Example 4 : Characteristics of the tobacco transformants producing bacterial OASTL-A targeted to the chloroplasts.

In leaf tissue cells of the transformants harboring the recombinant DNA molecule, the bacterial OASTL-A would be expected mainly in the chloroplasts. To verify the expected OASTL-A localization the immunocytodetection of exogenous OASTL-A in tobacco mesophyll cells was performed using polyclonal rabbit anti-OASTL-A IgG as primary antibodies, Cy3-conjugated sheep anti-rabbit IgG as secondary antibodies, and a Carl Zeiss fluorescence microscope equipped with an Olympus block filter for Rhodamine-Cy3 (absorption 450-490 nm, emission 510 nm). Results of this experiment proved that the targeting of OASTL-A to chloroplasts of the transformants was efficient since large amounts of the bacterial protein accumulated in this compartment. The results of immunodetection and immunocytolocalization of heterologously produced bacterial OASTL-A in transgenic tobacco (T1 generation, RCK line) are shown in Figure 4. The upper panel shows Western blot with anti-OASTL-A antibodies of the total cellular protein extracts (lane a) and the protein extracts from isolated chloroplasts of RCK tobacco (lane b). As a control, the total protein extract from the parental tobacco line was used (K-). The positions of protein standards (in kDa) are indicated. The lower panel shows pictures from the fluorescence microscope. The OASTL-A was immunolocalized using affinity purified rabbit anti-OASTL-A as primary antibodies and sheep anti-rabbit-Cy3 conjugate as secondary antibodies. The mesophyll cells of LA Burley 21 (parental line; left-hand slide) and of RCK transformant with targeted accumulation of the mature protein to the chloroplasts (right-handed slide) are shown. Careful visual analysis of the T1 progeny of primary transformants revealed phenotypic differences between them and the parental wild-type line, namely a slight bleaching of the leaves in the seedlings of the transformants; in some individual plants of the transgenic lines, similar symptoms could be observed during the entire plant development. Apart from this point, no striking differences in the growth characteristics nor morphology of the transgenic plants were observed ; all of the plants produced normal flowers and mature seeds.

Example 5 : Enzymatic assays on protein extracts from tobacco tissues and plants producing bacterial OASTL-A.

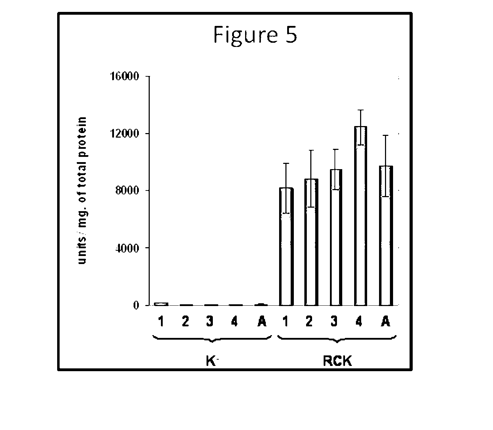

Functionality of the *cysK* transgene was analyzed at the protein level by assaying OASTL activity in several of the regenerated lines. The enzymatic assays were performed with two individuals of the same transgenic line, using leaf tissues collected from four successive leaves starting from the plant apex. As expected, the chloroplastic OASTL overproducer had highly increased activity of the enzyme (in average up to ∼ 240-fold in comparison to the parental LA Burley 21 line). However, differences between leaves from a same plants were observed. There was a positive correlation between OASTL activity and leaf age. In Figure 5, there is shown OASTL activity in leaf tissues of transgenic T1 individuals (RCK) or a parental line (K-) at a later stage of vegetative development (18-week-old). OASTL activity was assayed in four successive leaves from the plant apex as following : 1- budding leaf, 2- developing leaf, 3- mature leaf, 4- senescing leaf ; "A" is an average (n=8) of the enzymatic activity measured in leaves. Measurements were performed using the same procedure as described in Figure 3, in two individuals of the same line. One OASTL unit is defined as the amount of enzyme which catalyzes the formation of 1 nmole of cysteine per min in the conditions of the assay. Generally, all leaves of the transgenics deliver large amounts of the active exogenous OASTL-A and the plants can provide reliably high levels of the bacterial OASTL enzyme during at least their entire vegetative stage of growth.

Example 6 : Use of protein extracts and protein fractions derived from plant materials producing bacterial OASTL-A.

It's worth mentioning that bacterial OASTL-A is a robust enzyme, that do not require special care upon handling; it remains quite highly stable in chilling conditions or at room temperature, and its activity could be maintained upon several freezing / thawing cycles. In our hands, OASTL-A biosynthetic capacity in plant protein extracts yielded up to ∼ 10000 units per mg of total protein, i.e. ∼ 6 ng cysteine per minute per mg. FW. The high activity of the bacterial enzyme was also demonstrated in protein extracts from the leaf tissues of the transgenic plants by assaying protein fractions resolved in a colorless native polyacrylamide gel electrophoresis (Schagger *et al.,* 1994). The enzyme of our interest being sequestrated into the gel proved its ability to achieve production of cysteine from OAS and sulfite since the previous compound, that readily diffused through the gel, could be detected using a azo-dye-chromophore according to a non-invasive method for assaying *O-*acetylserine sulfhydrylase (Becker *et al*., 1969). Once after its biosynthesis, cysteine should be separated from the other compounds released from the reaction, for instance performing purification of the amino acid residue by the means of HPLC.

### Reference signs list

**References**

BECKER M., KREDICH N., TOMKINS G. (1969). The purification and characterization of O-acetylserine sulfhydrylase-A from Salmonella typhimurium. J. Biol. Chem., 244 : 2418-2427

CARRINGTON J., FREED D., LEINICKE A. (1991). Bipartite signal sequence mediates nuclear translocation of the plant potyviral NIa protein. Plant Cell, 3 : 953-962

GAITONDE M. (1967). A spectrophotometric method for the direct determination of cysteine in the presence of other naturally occurring amino acids. Biochem. J., 104 : 627-633

HELLENS R., EDWARDS E., LEYLAND N., BEAN S., MULLINEAUX P. (2000). pGreen: a versatile and flexible binary Ti vector for Agrobacterium-mediated plant transformation. PlantMol. Biol., 42 : 819-832

HOEKEMA A., HIRSCH P., HOOYKAAS P., SCHILPEROORT R. (1983). A binary vector strategy based on separation of vir- and T-region of the Agrobacteriumtumefaciens Ti-plasmid. Nature, 303 : 179-180

JAGURA-BURDZY G., HULANICKA D. (1981). Use of gene fusions to study expression of cysB, the regulatory gene of the cysteine regulon. J. Bacteriol., 147 : 744-751

LEGG P., COLLINS G., LITTON C. (1970). Registration of LA Burley 21 tobacco germplasm. Crop Sci., 10 : 212

MEIER I., CALLAN K., FLEMING A., MANZARA T., GRUISSEM W. (1995). Organ-specific differential regulation of a RBCS promoter subfamily in tomato. Plant Physiol., 107: 1105 -1118

SCHAGGER H., CRAMER W., VONJAGOW G. (1994). Analysis of molecular masses and oligomeric states of protein complexes by blue native electrophoresis and isolation of membrane protein complexes by two-dimensional native electrophoresis. Anal. Biochem., 217 : 220-230

WARRILOW A., HAWKESFORD M. (1998). Separation, subcellular location and influence of sulphur nutrition on isoforms of cysteine synthase in spinach. J. Exp. Bot., 49 : 1625-1636

## Claims

1. A recombinant DNA molecule comprising a nucleic acid molecule encoding a protein having cysteine synthase activity (EC 2.5.1.47) from a prokaryote or archaebacteria, wherein said nucleic acid molecule is operably linked to regulatory elements allowing the expression of said the nucleic acid molecule in a plant cell.

2. The recombinant DNA molecule of claim 1, wherein said protein having cysteine synthase activity is called either of the followings - cysteine synthase (CS), *O*-acetylserine(thiol)lyase (OASTL), *O*-acetylserine sulfhydrylase (OASS).

3. The recombinant DNA molecule of claim 1 or 2, wherein nucleic acid molecule is operably linked to a nucleotide sequence encoding a transit peptide capable of directing the protein into a desired cellular compartment.

4. The recombinant DNA molecule of claim 3, wherein said cellular compartment is a plastid.

5. The recombinant DNA molecule of any one of claims 1 to 4, wherein said regulatory elements comprise a promoter active in a plant cell,

6. The recombinant DNA molecule of claim 5, wherein said promoter is inducible, constitutively expressed and/or is a cell, tissue or organ specific promoter.

7. The recombinant DNA molecule of claim 6, wherein said promoter is tuber specific, seed-specific, endosperm-specific, embryo-specific, or phloem specific.

8. The recombinant DNA molecule of claim 1, comprising a nucleic acid molecule encoding a protein having cysteine synthase activity derived either from *Escherichia colicysK* gene or its homologues from other bacteria, is called either of the followings *Escherichia colicysK* gene or its homology from other bacteria's, wherein said nucleic acid molecule is linked to regulatory elements allowing the expression of said the nucleic acid molecule in to a plant cell, preferably in to chloroplast.

9. A vector containing a plant expression cassette comprising at least one recombinant DNA molecule of any one of claims 1 to 8.

10. A transgenic plant cell or plant cell culture comprising stably integrated into its genome at least one recombinant DNA molecule of any one of claims 1 to 8 or a vector of claim 9.

11. The transgenic plant cell or plant cell culture as defined in claim 10, wherein the amount of functionally active cysteine synthase is several-fold increased while comparing with that in plant cell breeding of the wild-type cultivar.

12. The transgenic plant cell or plant cell culture or plant tissue comprising transiently or stably integrated into its genome at least one recombinant DNA molecule of any one of claims 1 to 8, or a vector of claim 9, or a transgenic plant cell as defined in claim 10 or 11.

13. The transgenic plant or plant tissue of claim 12, wherein the amount of functionally active cysteine synthase is several-fold increased while comparing with that in plant of the wild-type cultivar.

14. Use of recombinant DNA molecule of any one of claims 1 to 8 for production of transgenic plant material as defined in claims 10 to 13.

15. Use of protein issue from any one of claims 1 to 8, or a vector of claim 9, or a transgenic plant cell as defined in claim 10 or 13 for production of L-cysteine and/or biochemical compounds containing or derived from L-cysteine, as well as for production of additives and/or ingredients containing the said biomolecules.
